# EUROPEAN PATENT APPLICATION

(11) **EP 4 674 370 A1**
(43) Date of publication of application: **07.01.2026**
(21) Application number: 24929403.4
(22) Date of filing: 06.12.2024
(51) Int. Cl.: A61B 34/00

(54) **MULTI-DEGREE OF FREEDOM SURGICAL DEVICE AND DRIVING METHOD THEREFOR**

(30) Priority: 03.05.2024 KR 20240059407
(71) Applicant: Curexo Inc., Seoul 05814 (KR)
(72) Inventor: KIM, Young Hwan, Songpa-gu, Seoul 05814 (KR); KIM, Yeong Seok, Songpa-gu, Seoul 05814 (KR); KIM, Si Jin, Songpa-gu, Seoul 05814 (KR)
(74) Representative: Lavoix
(86) International application number: PCT/KR2024/019915
(87) International publication number: WO 2025/230075

(57) **Abstract**

A surgical device and a method of controlling the surgical device are described. The surgical device includes a mounting plate placed on a plane parallel to both a first axis along which a tool extends and a second axis crossing the first axis, a first actuator configured to generate a first motion of the mounting plate in a direction of the second axis with the mounting plate mounted thereon, a second actuator configured to generate a rotational force to rotate the first actuator around a third axis orthogonal to the first and second axes to generate a second motion of the mounting plate, and a third actuator coupled to the second actuator to transfer the rotational force from the second actuator to the first actuator and generate a third motion by rotating the mounting plate around a rotation axis in the direction of the second axis.

## Description

### Technical Field

The present disclosure relates to a multi-degree-of-freedom surgical device used for joint surgery or the like, and a method of controlling the multi-degree-of-freedom surgical device, and more particularly, to a surgical device for aligning or positioning a surgical end-effector by three degrees of freedom or more and a method controlling the surgical device.

### Background Art

Joint damage caused by various reasons is accompanied by deformation and functional loss as well as pain. Artificial joint surgery may be selected when a nonsurgical method or even an initial surgical treatment by osteotomy or the like for a damaged joint is not effective.

Artificial joint surgery, which is one type of surgical operation, is performed by inserting an artificial replacement into a joint part, and for this, a portable saw driver or drill driver is used as a surgical device having a drill-type or saw-type end-effector, etc.

The saw driver is used to partially cut a bone of a joint part, and the drill driver is used to form a tunnel (hole) or fix a pin in a bone. For example, before a bone is cut by the saw driver, a tunnel is formed by the drill driver for insertion of a surgical pin, and the surgical pin is fixed therein, and in certain cases, the drill used to form the tunnel may remain as the pin.

During surgery, a stiff and damaged part is removed using a saw driver or the like from damaged joints, such as femur, tibia, or patella, and an artificial substitution, such as a finely manufactured artificial joint, is inserted and fixed into this surgical site.

To normally insert the artificial replacement into the surgical site, the position and direction of an end-effector need to be accurately maintained with respect to a surgical tube, so as to cut bone according to a pre-set surgical plan.

Though there is support of a so-called surgical navigation system, an end-effector of a previous low degree of freedom, for example, two degrees of freedom, cannot cope sufficiently well with a limited surgical space and restricted postures of a surgeon.

In this respect, it may be highly useful to develop a high degree of freedom surgical device, according to which a surgeon may have relatively increased freedom of posture, and a position and direction of an end-effector may match a corresponding surgical site according to a pre-set surgical plan.

### Disclosure of Invention

### Technical Problem

The present disclosure provides a surgical device having a high degree of freedom.

The present disclosure provides a surgical device for aligning and positioning an end-effector at a desired surgical position, regardless of a posture of a surgeon, and a method of controlling the surgical device.

### Solution to Problem

A surgical device according to one or more embodiments includes:
a mounting plate on which an end-effector comprising a surgical tool extending in a first direction may be mounted, the mounting plate being placed on a plane parallel to both a first axis in the first direction and a second axis in a second direction, the second direction crossing the first direction;
a first actuator on which the mounting plate is mounted and which generates a first motion of the mounting plate in a second direction crossing the first direction;
a second actuator configured to generate a rotational force to rotate the first actuator around a third axis in a third direction orthogonal to the first axis and the second axis, and to cause a second motion of the mounting plate; and
a third actuator coupled to the second actuator and configured to transmit the rotational force from the second actuator to the first actuator and to cause a third motion of the mounting plate by rotating the mounting plate around a rotation axis in the second direction.

In the surgical device according to one or more embodiments,
the first actuator may include: a first plate on which the mounting plate is installed to be reciprocally movable in the second direction; a first motor configured to drive the mounting plate; and a first power transmission unit configured to transmit power of the first motor to operate the mounting plate.

In the surgical device according to one or more embodiments,
the first power transmission unit may include: a first conversion unit configured to convert rotation of the first motor into a linear reciprocating movement; and a first operating rod configured to transmit a linear movement from the first conversion unit as a linear movement of the mounting plate.

In the surgical device according to one or more embodiments,
the linear movement from the first conversion unit may occur in a direction crossing a plane of the mounting plate, and the first operating rod may be hinge-coupled to the mounting plate to convert the linear movement from the first conversion unit into the movement of the mounting plate.

In the surgical device according to one or more embodiments,
the first conversion unit may include a first linear mover configured to perform the linear reciprocating movement by rotation of the first motor, and the first operating rod may be hinge-coupled to the mounting plate and the first linear mover.

In the surgical device according to one or more embodiments,
the first, second, and third actuators may be disposed within a housing that protects the first, second and third actuators, and the second actuator may include: a second plate fixed in position with respect to the housing; a second motor configured to generate rotational force to rotate the first actuator; and a second motor mount configured to support the second motor.

In the surgical device according to one or more embodiments,
the third actuator may include a third plate rotated by the second motor.

In the surgical device according to one or more embodiments,
the third actuator may include:
a third motor and a fourth motor configured to generate rotational forces to generate a third motion of the mounting plate; and
a second power transmission unit configured to generate the third motion by using respective rotations of the third motor and the fourth motor.

In the surgical device according to one or more embodiments,
the second power transmission unit may include:
a second conversion unit and a third conversion unit configured to convert the respective rotations of the third motor and the fourth motor into linear reciprocating movement; and
a third operating rod and a fourth operating rod configured to transmit respective linear movements of the second conversion unit and the third conversion unit to the mounting plate to generate the third motion.

In the surgical device according to one or more embodiments,
the third actuator may be configured such that the second conversion unit and the third conversion unit of the second power transmission unit move a same distance at a same time, to generate a fourth motion in which the mounting plate performs translational movement in the third direction without a change in inclination.

A method of controlling a surgical device, according to one or more embodiments, includes:
mounting an end-effector having a tool arranged in a first direction on a mounting plate placed on a plane parallel to both a first axis in the first direction and a second axis in a second direction crossing the first direction;
causing, by a first actuator, a first motion of the mounting plate in the second direction;
causing, by a second actuator, a second motion of the mounting plate by rotating the first actuator around a third axis in a third direction orthogonal to the first axis and the second axis; and

Causing, by a third actuator, a third motion of the mounting plate, the third actuator being coupled to the second actuator and configured to transmit a rotational force from the second actuator to the first actuator and rotate the mounting plate around a rotation axis in the second axis.

In the method of controlling a surgical device according to one or more embodiments,
a first plate provided in the first actuator may support the mounting plate to be reciprocally movable in the second direction, and a first power transmission unit provided in the first actuator may cause the first motion of the mounting plate.

In the method of controlling a surgical device according to one or more embodiments,
a first conversion unit provided in the first power transmission unit may convert rotation of a first motor into linear reciprocating movement, and a first operating rod connected to the first conversion unit may transmit a linear movement from the first conversion unit to the mounting plate.

In the method of controlling a surgical device according to one or more embodiments,
the first conversion unit may generate a linear movement in a direction crossing a plane of the mounting plate, and the first operating rod may be hinge-coupled to the mounting plate to convert the linear movement from the first conversion unit into the movement of the mounting plate.

In the method of controlling a surgical device according to one or more embodiments,
a housing may protect the first, second, and third actuators provided within the housing, and
a second plate provided in the second actuator may be fixed in position with respect to the housing and supports the first, second, and third actuators as a whole.

In the method of controlling a surgical device according to one or more embodiments,
the third actuator may transmit a rotational operation from the second actuator to the mounting plate to cause the second motion, and may cause the third motion by using a second conversion unit and a third conversion unit that convert respective rotations of a third motor and a fourth motor into linear reciprocating movement.

In the method of controlling a surgical device according to one or more embodiments,
the third actuator may generate a fourth motion, in which the mounting plate performs translational movement in the third direction without a change in inclination, by using same-distance movements of the second conversion unit and the third conversion unit.

### Brief Description of Drawings

FIG. 1 is a schematic perspective view of an end-effector type surgical device as a surgical device according to an embodiment of the present disclosure.
FIG. 2 is a schematic perspective view of a driver of a surgical device, from which an end-effector is removed, according to an embodiment of the present disclosure.
FIG. 3 is a schematic perspective view of a surgical device, from which a housing is removed, according to an embodiment of the present disclosure.
FIG. 4 is a partially exploded perspective view of an end-effector of a surgical device and an actuator assembly of a driver configured to drive the end-effector, according to an embodiment of the present disclosure.
FIG. 5 is a partial cross-sectional view according to another embodiment, which more specifically illustrates a coupling structure of a second operating rod in a surgical device according to an embodiment of the present disclosure.
FIG. 6 is a partial perspective view of an actuator assembly with a housing and an end-effector removed, in a surgical device according to an embodiment of the present disclosure.
FIG. 7 is a schematic perspective view of the actuator assembly viewed from a different direction from the direction of FIG. 6 in a surgical device according to an embodiment of the present disclosure.
FIG. 8 illustrates a translational movement or shifting movement of a mounting plate and a corresponding posture or position of an end-effector in a surgical device according to an embodiment of the present disclosure.
FIG. 9 illustrates results of a rotational (yawing) motion of a mounting plate and resulting postures or positions of an end-effector in a surgical device according to one embodiment of the present disclosure, and
FIG. 10 illustrates a third motion, which is a pitching movement, and a fourth motion, which is a vertical rising and falling or translational movement, of a mounting plate in a surgical device according to an embodiment of the present disclosure.

### Mode for the Invention

Hereinafter, preferred embodiments of the present inventive concept are described in detail with reference to the accompanying drawings. However, the embodiments of the present inventive concept may be modified into various other forms, and the scope of the present inventive concept shall not be construed as being limited to the embodiments described below in detail. It is desirable that the embodiments of the present inventive concept be interpreted as being provided to more fully explain the present inventive concept to one of ordinary skill in the art. The same signs denote the same elements throughout. Furthermore, various elements and areas in the drawings are schematically drawn. Therefore, the present inventive concept is not limited by the relative sizes or distances illustrated in the accompanying drawings.

Terms such as first, second, etc. may be used to describe various components, but the components are not defined by these terms. The terms are used only for distinguishing one element from another element. For example, without deviating from the scope of the claims of the present inventive concept, a first element may be referred to as a second element, and vice versa.

The terms used in the present application are used only for describing particular embodiments and are not intended to limit the present inventive concept. A singular expression may include a plural expression, unless an apparently different meaning is indicated in the context. With respect to the present application, it will be further understood that the expressions "comprises" and "comprising" used herein specify the presence of stated features, integers, steps, operations, members, components, and/or groups thereof, but do not preclude the presence or addition of one or more other features, integers, operations, members, components, and/or groups thereof.

Unless otherwise defined, all of the terms used herein including technical terms and scientific terms have the same meaning as the meaning commonly understood by one of ordinary skill in the art. Also, the terms commonly used and having the meanings as defined in dictionaries shall be understood to have consistent meanings with the corresponding terms in the context of relevant arts, and unless explicitly defined so herein, the meanings of the terms shall not be understood to be excessively formal.

Hereinafter, a surgical device and a method of the same are described according to one or more embodiments.

The surgical device according to the present disclosure basically includes the following components a mounting plate, a first actuator, a second actuator, and a third actuator.

The mounting plate is mounted with an end-effector comprising a surgical tool extending in a first direction, and is placed on a plane parallel to both a first axis in the first direction and a second axis in a second direction crossing the first direction.

The first actuator is mounted with the mounting plate and generates a first motion M1 of the mounting plate in the second direction.

The second actuator generates a rotational force to rotate the first actuator around a third axis in a third direction orthogonal to the first axis and the second axis, to thereby generate a second motion M2 of the mounting plate.

The third actuator is coupled to the second actuator, transfers the rotational force from the second actuator to the first actuator, and generates a third motion M3 by rotating the mounting plate around a rotation axis in the second direction.

The actuators include a power transmission unit, that is, a powertrain system configured to transmit rotational power from a motor as rotational or linear power. The first actuator includes a first plate on which the mounting plate is installed to be reciprocally movable in the second direction, a first motor configured to drive the mounting plate, and a first power transmission unit configured to transfer power of the first motor to operate the mounting plate.

The first power transmission unit includes a first conversion unit configured to convert a rotational movement of the first motor into a linear reciprocating movement, and a first operating rod configured to transmit a linear movement from the first conversion unit as a linear movement of the mounting plate.

The first conversion unit includes a first linear mover configured to perform a linear reciprocating movement by a rotation of the first motor, and the first operating rod is hinge-coupled to the mounting plate and the first linear mover.

The third actuator includes a third motor and a fourth motor configured to generate rotational forces to generate a third motion of the mounting plate, and a second power transmission unit configured to generate the third motion by using respective rotations of the third motor and the fourth motor.

The second power transmission unit includes a second conversion unit and a third conversion unit configured to convert respective rotations of the third motor and the fourth motor into linear reciprocating movements, and a third operating rod and a fourth operating rod configured to transfer the linear movement of each of the second conversion unit and the third conversion unit to the mounting plate to generate the third motion.

The second conversion unit and the third conversion unit have structures based on a concept similar to that of the first conversion unit. That is, a second linear mover and a third linear mover, which perform linear reciprocating movements by rotations of the third motor and the fourth motor, are provided, and the third operating rod and the fourth operating rod are respectively included to the second linear mover and the third linear mover. This structure may be clearly understood through the corresponding description provided with reference to the drawings.

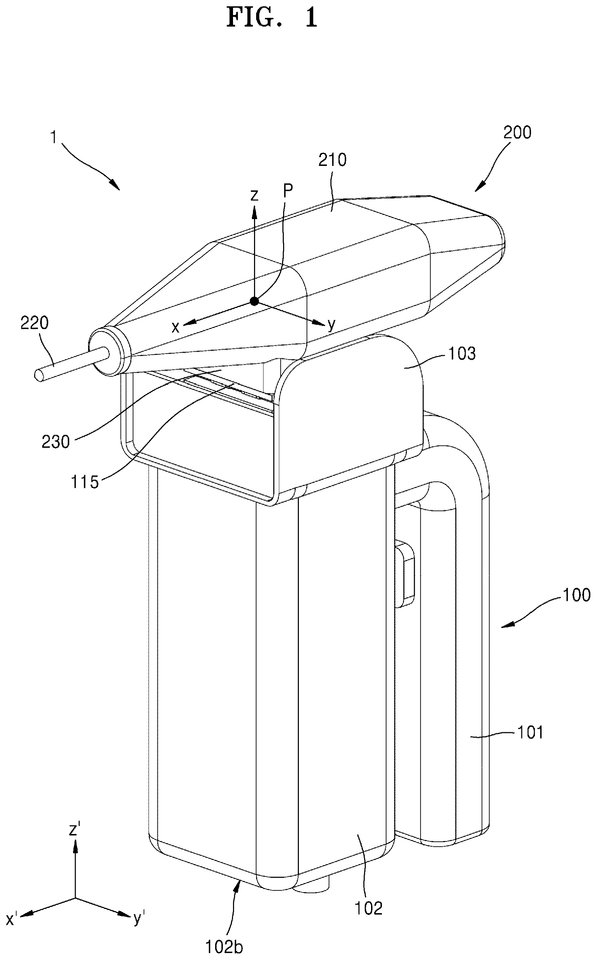

FIG. 1 is a schematic perspective view of an end-effector type surgical device according to an embodiment of the present disclosure.

Referring to FIG. 1, an end-effector type surgical device 1 (hereinafter, the surgical device) may include an end-effector 200 and an end-effector driver 100 (hereinafter, a driver) on which the end-effector 200 may be mounted.

The driver 100 may include a housing 102 having a grip 101, a mounting plate 115 on which the end-effector 200 is mounted and which is exposed above the housing 102, and a pair of protection fences 103 configured to protect the mounting plate 115.

The end-effector 200 may be provided with a surgical tool 220, such as a saw or drill, a driving unit for driving the surgical tool 220, and a case 210 accommodating the driving unit including, for example, driving motors, and a mounting foot 230, which is coupled to the mounting plate 115, may be provided at a lower portion of the case 210 in this embodiment. The coupling of the mounting foot 230 to the mounting plate 115 may be achieved by a fixed coupling structure or a fastening structure, both of which are detachable.

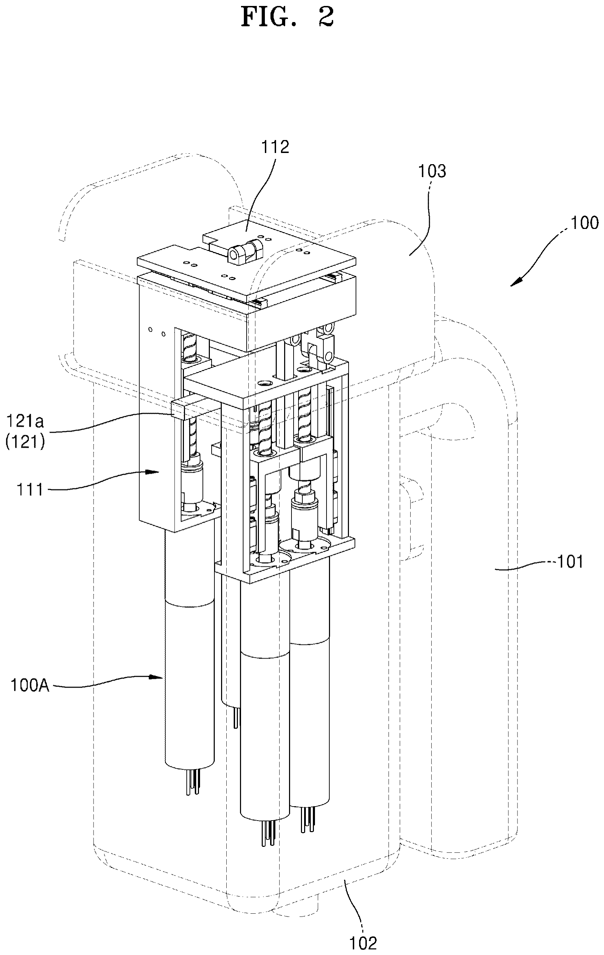

FIG. 2 is a schematic perspective view of the driver 100 in the surgical device shown in FIG. 1, in which the end-effector 200 is removed, and the housing 102 and the grip 101 are illustrated in dashed lines to expose an actuator assembly 100A inside the housing 102.

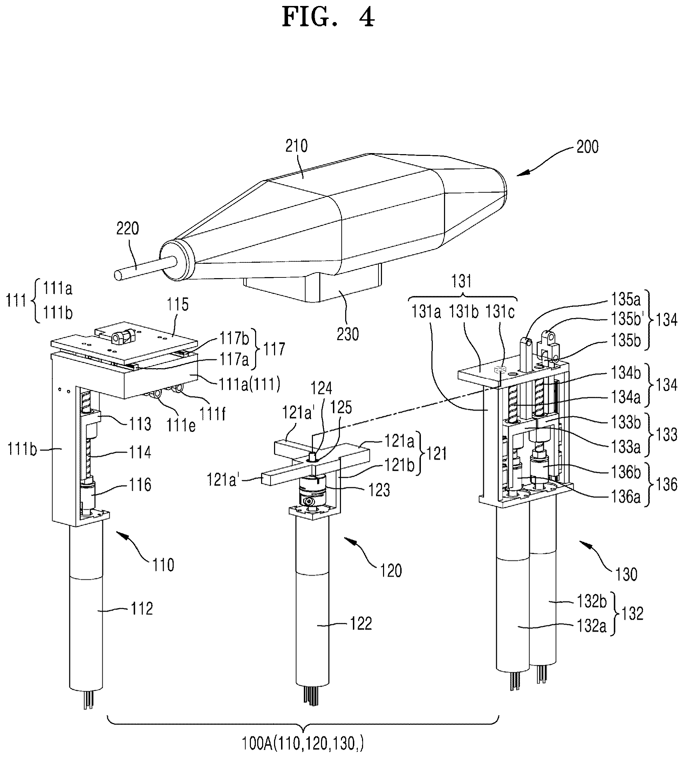

The housing 102, which has the grip 101 formed on one side thereof, has a box shape with an open top corresponding to the end-effector 200. An actuator assembly 100A configured to operate the mounting plate 115 may be fixed inside the housing 102. The actuator assembly 100A includes a plurality of components configured to move the mounting plate 115 in multi-degree of freedom, and these components are directly or indirectly supported by a second plate 121a that serves as a main frame firmly fixed to an inner wall of the housing 102. As will be described in detail later, the main frame, that is, the second plate 121a, serves as a base structure for fixing the actuator assembly 100A inside the housing 102 and is a component of the second bracket 121, which will be described in detail with reference to FIG. 4.

Returning to FIG. 1, in the surgical device 1 having the structure as described above, the end-effector 200, in a state of being mounted on the driver 100, is driven by the driver 100 to perform various movements with three or four degrees of freedom in a three-axis direction X-Y-Z, including an X-axis in a first direction, a Y-axis in a second direction orthogonal or crossing the first direction, and a Z-axis in a third direction orthogonal or crossing both the first and second directions.

In FIG. 1, the driver 100 maintains the end-effector 200 in a neutral posture at an operation center p, and based on this state, an X-Y-Z coordinate system and an X'-Y'-Z' coordinate system with a different origin coordinate may be defined.

In the X'-Y'-Z' coordinate system, the X'-Y' plane is the plane on which the bottom 102b of the housing 102 of the driver 100 is placed and is parallel to the X-Y plane passing through the operation center p of the end-effector 200. The Z'-axis orthogonal to the X-Y plane extends in the direction in which the housing 102 of the driver 100 is vertically aligned and is parallel to the Z-axis passing through the operation center p of the end-effector 200, and the X'-axis is parallel to the X-axis along which the surgical tool 220 extends.

The origin coordinate of the X-Y-Z coordinate system is located at the operation center p of the end-effector 200, and thus movement operations of the end-effector 200 may be described with reference to the origin coordinate.

Describing movement operations, that is, motions, of the end-effector 200 using the X-Y-Z coordinate system as described above is intended to facilitate easier understanding of each of various motions or a complex motion in which these motions are combined, according to the present disclosure.

According to various movement operations of the driver 100, the end-effector 200, to which the surgical tool 220 is mounted, is capable of performing various movements in the three-axis coordinate system. Terms used in definitions of movements of an aircraft or a vehicle may be partially or entirely used in describing movements of the end-effector 200.

Various movement operations of the end-effector 200 may include the first motion M1 of shifting or translation, the second motion M2 of yawing, and a third motion M3 of pitching, and additionally may include a fourth motion M4 of vertical elevation or bouncing in a vertical direction of the Z-axis.

The translational operation refers to a state in which the end-effector, moved in one direction by the first motion M1 or the fourth motion M4, changes its position laterally along the Y-axis or vertically along the Z-axis without a change in posture.

Each of the movements in the X, Y, or Z direction may be aligned with or parallel to the X, Y, or Z axis, or may be inclined slightly while having the same directionality as the axes.

These various types of motions may be performed in a combined manner according to the present disclosure. These various types of motions are generated by the driver 100, and will be described in detail hereinafter.

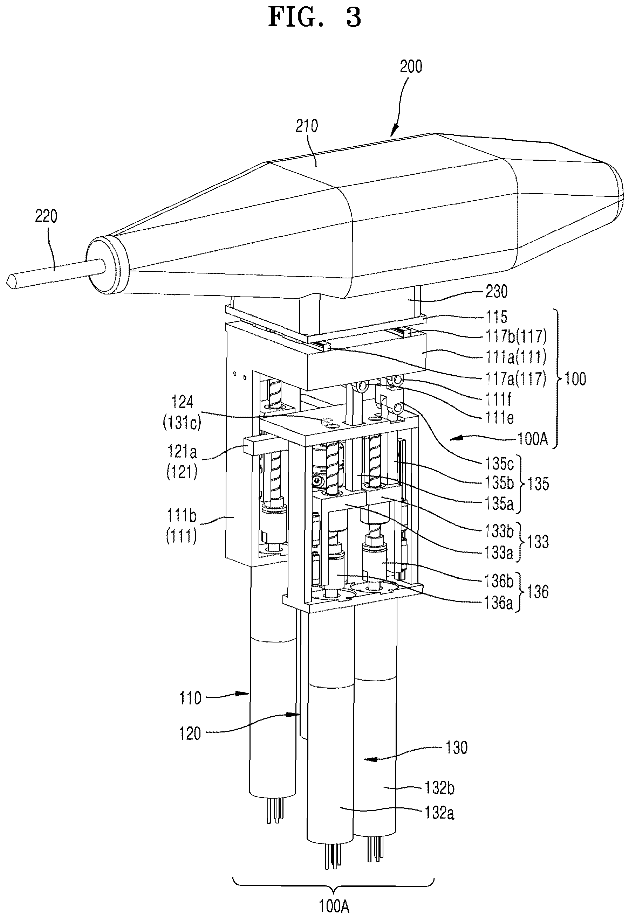

FIG. 3 is a schematic perspective view of the surgical device 1 with the housing 102 (see FIGS. 1 and 2) removed, and FIG. 4 is a partially exploded perspective view of an actuator assembly 100A of the end-effector 200 and the driver 100 for driving the same.

Referring to FIG. 3, the end-effector 200 is coupled to the mounting plate 115 of the driver 100 by the mounting foot 230 provided at a lower side of the end-effector 200, and the mounting plate 115 may be coupled to the actuator assembly 100A.

The mounting plate 115 ultimately causes all movements of the end-effector 200, and all these movements occur as relative movements with respect to the second plate 121a. As described above, the second plate 121a may be fixed to the housing 102 of the driver 100, fixes the actuator assembly 100A within the housing 102, and also serves as a component of the second bracket 121 that supports a second actuator 120 to be described later.

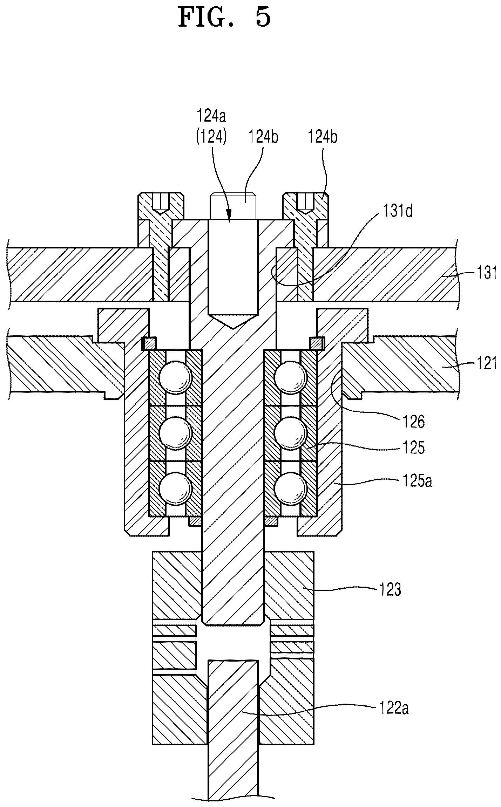

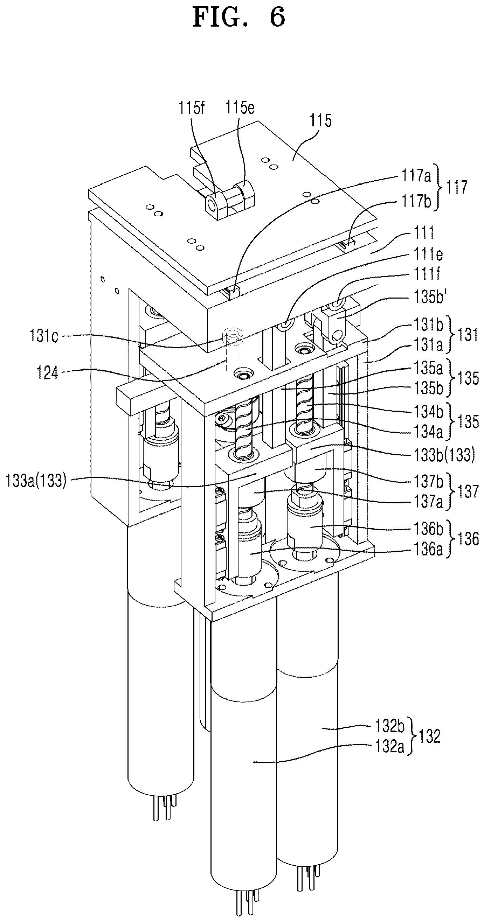

In the following description of the actuator assembly 100A, FIG. 4 is mainly referred to together with FIG. 3. However, FIGS. 5 and 6 are partially referred to for better understanding, but will be separately described again.

Referring to FIGS. 3 and 4 together, the actuator assembly 100A configured to operate the end-effector 200 is a combination in which a first actuator 110, the second actuator 120, and a third actuator 130 are operably coupled to one another.

The first actuator 110 includes a first bracket 111 configured to support the mounting plate 115 to be shiftable laterally within a preset working distance by a linear rail unit 117 including two slide rails 117a and 117b. The first bracket 111 includes a first plate 111a and a first motor mount 111b, which are integrally assembled from a plurality of components or formed as a single body.

The linear rail unit 117 may be installed on the first plate 111a, and a first motor 112, which generates lateral shift movement of the mounting plate 115, is mounted on the first motor mount 111b. A rotation shaft of the first motor 112 may be connected to a linear feed screw 114 through a first coupler 116, and the linear feed screw 114 may be screw-coupled to a threaded mover 114a (see FIG. 6) coupled to a first linear mover 113a that moves the operating rod assembly 119 (see FIG. 6), thereby reciprocating the first linear mover 113a within the operation range. Here, the first linear mover 113a and the first motor mount 111b may have portions parallel to each other, and between these parallel portions, a guide rail structure 118 (see FIG. 6) may be installed to guide the linear reciprocating movement of the first linear mover 113a in the extension direction of the linear feed screw 114.

The second actuator 120 may include a second motor 122 configured to generate a rotation (yawing or turning) movement of the mounting plate 115 and the second bracket 121 that supports the second motor 122. The second motor 122 rotates the second plate 121a within a predefined angular range to cause the yawing operation of the mounting plate 115, which is mounted to be laterally shiftable on the first plate 111a.

The second bracket 121 may include a second motor mount 121b on which the second motor 122 may be mounted and the second plate 121a, which serves as a main frame as described above. The second motor mount 121b may be integrally coupled to the second plate 121a, and according to another embodiment, the second bracket 121 may be formed as a single body with the second plate 121a.

The second plate 121a or the second bracket 121 including the second plate 121a serves, as described above, as a basic support structure for supporting the second actuator 120 and fixing the entire actuator assembly 100A within the housing 102, and may be varied in shape depending on the structural design.

In the present embodiment, the second plate 121a has a shape similar to an "L" with two extension portions, and the extension portions are fixed ends 121a' fixed to internal components of the housing 102, such as an inner wall. The second plate 121a may be fixed in position with respect to the housing 102 and serves as the basic support structure of the entire actuator assembly 100A.

Meanwhile, the rotation shaft of the second motor 122 mounted on the second bracket 121 may be directly connected to a second coupler 123, and the second coupler 123 is directly connected to a second operating rod 124 of a rotary type that rotates the third actuator 130. The second operating rod 124 may pass through the second plate 121a and may be firmly fixed to a shaft fixing portion 131c formed on a lower surface of a third bracket 131 of the third actuator 130. The second plate 121a, through which the second operating rod 124 passes, accommodates a bearing 125 (see FIG. 6) that assists rotation of the second operating rod 124 within a preset rotational range, together with components that support the bearing.

FIG. 5 is a partially extracted sectional view according to another embodiment that details a coupling structure of the second operating rod 124.

Referring to FIG. 5, a second operating rod 124a, which has a different form from the above-described second operating rod 124, has a pillar portion connected to the second coupler 123 and a head portion overlapping an upper surface circumference of a through-hole 131d formed in a third plate 131b. The head portion of the second operating rod 124a may be fixed to the third plate 131b by a fixing bolt 124b, and a waist portion of the second operating rod 124a may be supported for rotation by a bearing 125 within a bearing housing 125a installed in a through-hole 126 of the second plate 121a, as in the above-described embodiment.

According to this structure, the second actuator 120 rotates as a whole within a preset angular range by rotation of the second motor 122, thereby causing the yawing (turning) operation of the above-described end-effector 200.

FIG. 6 is a partial perspective view of the actuator assembly in a state where the housing and the end-effector are removed.

Referring to FIG. 6, the third actuator 130 includes two motors, that is, a third motor 132a and a fourth motor 132b, and a third bracket 131 supporting the motors. Here, the third bracket 131 may include a third motor mount 131a that supports the third motor 132a and the fourth motor 132b side by side, and a third plate 131b that may be integrally coupled to the third motor mount 131a and was described in the description of the second actuator 120.

As described above, the third plate 131b may be rotated by the second motor 122, and accordingly, all elements of the third actuator 130 combined with the third plate 131b may rotate together. The third actuator 130 may be operably connected to the first plate 111a of the first bracket 111 by an operating rod structure 135 operated by each of the third motor 132a and the fourth motor 132b.

In detail, the operating rod structure 135 may include a third operating rod 135a operably connected to the third motor 132a and a fourth operating rod 135b operably connected to the fourth motor 132b.

Lower ends of the third operating rod 135a and the fourth operating rod 135b may be coupled to a second linear mover 133a and a third linear mover 133b, respectively, which reciprocate within an operation range of the preset working distance by the third motor 132a and the fourth motor 132b. Here, although not described in detail, the third and fourth couplers 136: 136a and 136b with the coupler structure as described above may be coupled to respective rotation shafts of the third motor 132a and the fourth motor 132b.

Upper portions of the third operating rod 135a and the fourth operating rod 135b may be hinge-coupled to allow pivoting, that is, relative rotation, with respect to the first plate 111a.

Specifically, the upper portion of the third operating rod 135a may be hinge-coupled to allow pivoting with respect to a first hinge portion 111e provided on the first plate 111a. The upper portion of the fourth operating rod 135b may be pivotably connected to the second hinge portion 111f provided on the first plate 111a through a fifth operating rod 135c. Here, the fifth operating rod 135c serves to allow an offset or mismatch between an end of the fourth operating rod 135b and the second hinge portion 111f caused by tilting operation of the first plate 111a, while transmitting a force from the fourth operating rod 135b to the first plate 111a through the second hinge portion 111f.

According to this pivoting structure of the third operating rod 135a and the fourth operating rod 135b, simultaneous reciprocating of the third operating rod 135a and the fourth operating rod 135b allows the first plate 111a to perform elevating or up-and-down translational movement (bouncing) in the vertical Z direction, and different operations between the third operating rod 135a and the fourth operating rod 135b, such as when the fourth operating rod 135b reciprocates over a relatively longer distance than the third operating rod 135a, or when the fourth operating rod 135b reciprocates while the third operating rod 135a is stopped, allow the first plate 111a to perform the pitching operation about the first hinge portion 111e. That is, the third actuator 130 may perform either a pitching operation or a vertical elevating operation, and may also perform a combined pitching-bouncing operation.

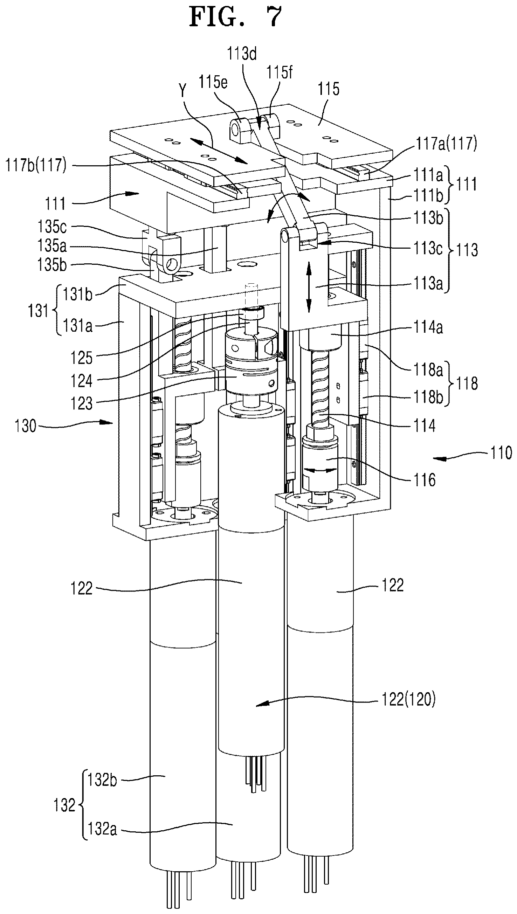

FIG. 7 is a schematic perspective view of the actuator assembly 100A viewed from a direction different from the direction of FIG. 6, showing a more detailed structure of the first actuator 110, and illustrating a state in which some components, such as the second plate 121a serving as the main frame, are removed for structural understanding.

Referring to FIG. 7, the mounting plate 115, which is supported to perform the lateral translational movement (shifting operation) within the preset working distance by a linear rail unit 117 including the two slide rails 117a and 117b on the first plate 111a, may be operated by an operating rod assembly 113 of the first actuator 110.

The operating rod assembly 113 includes a first linear mover 113a that reciprocates along the linear feed screw 114, and a first operating rod 113b of a rotary lever-type having both ends hinge-coupled to the first linear mover 113a and the mounting plate 115.

To this end, a third hinge portion 113c and a fourth hinge portion 113d are provided at both ends of the first operating rod 113b, such that the first operating rod 113b may be pivotable with respect to the first linear mover 113a and the mounting plate 115 through the third hinge portion 113c and the fourth hinge portion 113d. The third hinge portion 113c has a pin-coupling structure that engages with an end of the first operating rod 113b to allow relative movement, and the fourth hinge portion 113d is pivotably coupled to hinge members 115e and 115f provided on the mounting plate 115 through complementary engagement.

According to the above structure, rotation of the first motor 112 transmitted through the first coupler 116 causes linear movement of the first linear mover 113a, and the linear movement changes the posture of the first operating rod 113b, which is pivotally connected to the first linear mover 113a, thereby causing the mounting plate 115 to perform the translational movement or shifting in the Y-axis direction.

In the second actuator 120 that causes a rotational movement of the mounting plate 115 within the predefined angular range, the second operating rod 124, which is connected to the second motor 122 via the second coupler 123, may be firmly fixed to the shaft fixing portion 131c formed on a lower side of the third plate 131b of the third actuator 130. Reference numeral 125 in the drawing denotes the bearing that rotatably supports the second operating rod 124 with respect to the second bracket 121.

Meanwhile, the third actuator 130, driven by rotation of the second motor 122, may include the third bracket 131 having the third motor mount 131a and the third plate 131b. Here, the third operating rod 135a and the fourth operating rod 135b, driven by the third motor 132a and the fourth motor 132b respectively, may pass through respective through-holes formed in the third plate 131b and may be directly or indirectly connected to the first plate 111a.

Here, the upper end of the third operating rod 135a may be directly connected to the first plate 111a, and an upper end of the fourth operating rod 135b may be indirectly connected to the first plate 111a via the fifth operating rod 135c and a second hinge portion 111f provided on the first plate 111a. Here, the fifth operating rod 135c serves to allow an offset or mismatch between an end of the fourth operating rod 135b and the second hinge portion 111f caused by tilting operation of the first plate 111a, while transmitting a force from the fourth operating rod 135b to the first plate 111a through the second hinge portion 111f.

According to this pivoting structure of the third operating rod 135a and the fourth operating rod 135b, simultaneous reciprocating of the third operating rod 135a and the fourth operating rod 135b allows the first plate 111a to perform elevating or up-and-down translational movement (bouncing) in a single direction (Z direction), and different operations between the third operating rod 135a and the fourth operating rod 135b, such as when the fourth operating rod 135b reciprocates over a relatively longer distance than the third operating rod 135a, or when the fourth operating rod 135b reciprocates while the third operating rod 135a is stopped, allow the first plate 111a to perform tilting in one direction about the first hinge portion 111e, that is, the pitching operation. That is, the third actuator 130 may perform either the pitching operation or the vertical elevating operation, and may also perform the combined pitching-bouncing operation.

Hereinafter, various operations according to a multi-degree of freedom of the driver 100 (according to the present disclosure are described.

The actuator assembly 100A of the driver 100 according to the present disclosure may have the three actuators, operations of the actuators may be individually performed, and the operations of the actuators may be simultaneously performed to generate highly diverse and combined end-effector movements. Hereinafter, the operations are described with reference to the drawings.

### Operation of the First Actuator

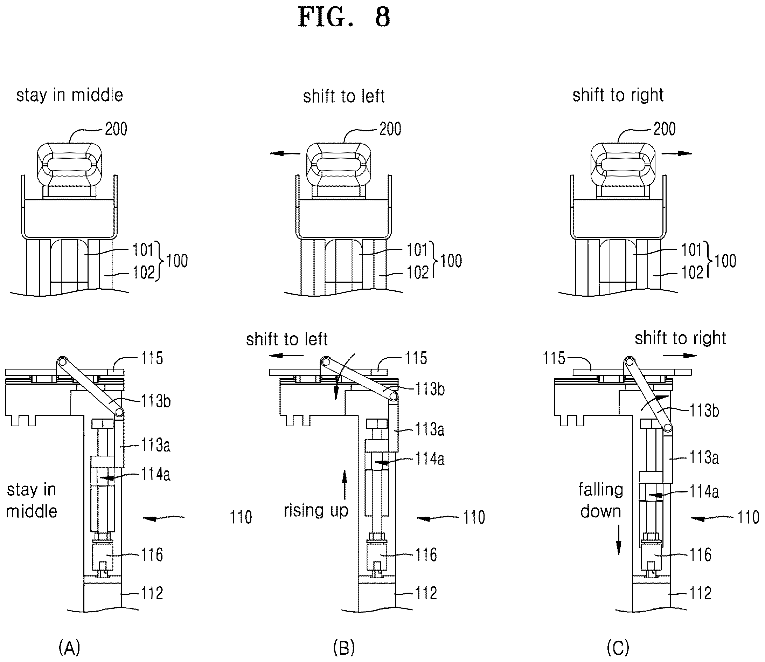

(A), (B), and (C) of FIG. 8 each shows a result of the translational movement or shifting operation, which is the first motion M1 of the mounting plate 115 through the first actuator 110, and a posture or a position of the end-effector 200 arising therefrom.

In FIG. 8, (A) illustrates a state in which the first actuator 110 stays in a middle position (initial posture), (B) illustrates a state in which the first motor 112 of the first actuator 110 operates so that the first linear mover 113a rises above the initial middle position (the middle position of an operating area), and (C) illustrates a state in which the first actuator 110 falls downward below the middle position.

Thus, in FIG. 8, in the state of (A), the end-effector 200 may stay in a middle position according to the middle position (initial posture) of the first actuator 110, in the state of (B), due to rising up of the first linear mover 113a, the first operating rod 113b of a operational lever-type ( 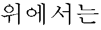 rotary level-type) may operate to push the mounting plate 115, and thus, the end-effector 200 thereabove may be shifted to the left side, and in the state of (C), the first actuator 110 may fall down below the middle position to pull the mounting plate 115, and thus, the end-effector 200 thereabove may be shifted to the right side.

### Operation of the Second Actuator

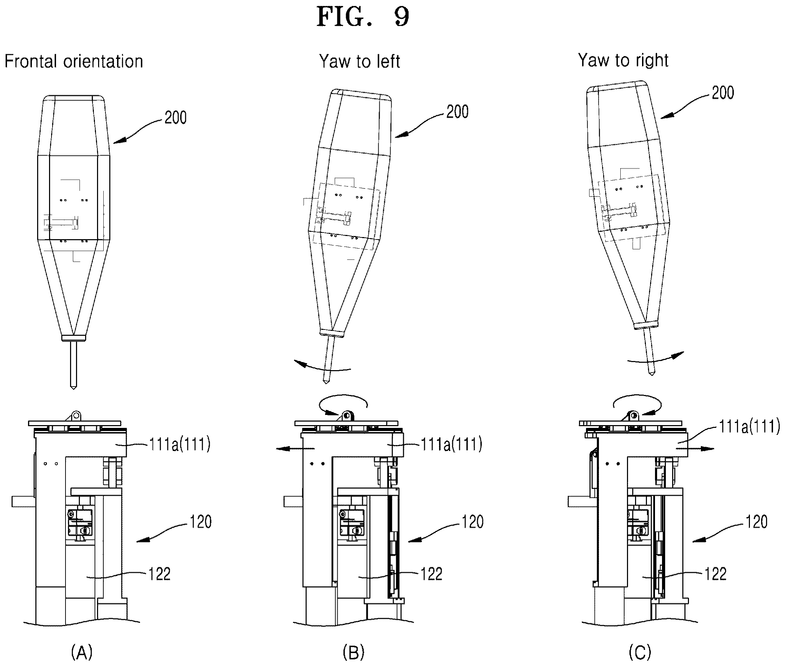

(A), (B), (C) of FIG. 9 each shows a result of the second motion M2, which is the yawing (turning) operation of the first plate 111a by the second actuator 120 and the posture of the end-effector 200 according to the result, respectively.

In FIG. 9, (A) shows a state in which the second actuator 120 stays in a middle position (initial posture), (B) shows a state in which the second motor 122 of the second actuator 120 operates to rotate the first plate 111a of the first bracket 111 in one direction (clockwise) at a predefined angle, and (C) shows a state in which the second motor 122 operates to rotate the first plate 111a of the first bracket 111 in the other direction (counterclockwise) at a predefined angle.

Thus, in FIG. 9, in the state of (A), the end-effector 200 may stay in the middle position toward the front side in the drawing according to the middle position (initial posture) of the second actuator 120, in the state of (B), the end-effector 200 may be yawed (turned) to the left side by a certain angle, and in the state of (C), the end-effector 200 may be yawed (turned) to the right side by a certain angle.

### Operation of the Third Actuator

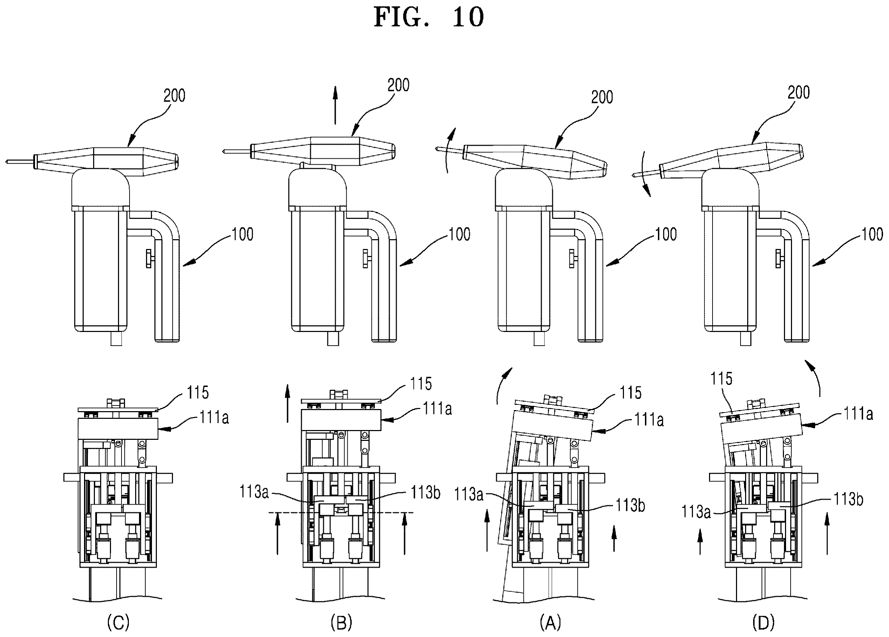

(A), (B), (C), and (D) of FIG. 10 each illustrates the third motion, which is the pitching operation, and the fourth motion, which is the vertical elevating or the translational movement, of the end-effector 200, and show the surgical tool 220 of the surgical device 1 facing the left side in the drawing. (A), (B), (C), and (D) of FIG. 10 each shows a result of the operation of the third motor 132a and the fourth motor 132b of the third actuator 130 that operates the first plate 111a of the first bracket 111, and a corresponding posture or position of the end-effector 200.

In FIG. 10, (A) illustrates a state where the third actuator 130 stays in an initial pose, and (B) shows a state where the second linear mover 133a and the third linear mover 133b rise by the same distance by simultaneous operation of the third motor 132a and the fourth motor 132b of the third actuator 130. In FIG. 10, (C) shows a state where the second linear mover 133a rises to a relatively greater height than the third linear mover 133b due to differential operation of the third motor 132a and the fourth motor 132b, and (D) shows a state opposite to that of (C), where the third linear mover 133b rises to a relatively greater height than the second linear mover 133a due to differential operation of the third motor 132a and the fourth motor 132b.

Accordingly, in FIG. 10, in the state of (B), the first plate 111a performs the translational movement as the fourth motion vertically and in parallel with the plane of the first plate 111a at a higher position compared to the state of (A), and thus, the first plate 111a vertically rises without a change in the inclination.

In FIG. 10, in the state of (C), the first plate 111a does not perform the vertical translation as in the state of (B), and a left edge of the first plate 111a is higher than a right edge thereof, and thus, the plane of the first plate 111a is tilted to the left.

In FIG. 10, in the state of (D), opposite to the state of (C), a right edge of the first plate 111a is higher than a left edge thereof in the drawing, and thus, a plane of the first plate 111a is tilted to the right.

Accordingly, in FIG. 10, the end-effector 200 moves straight upward in the vertical direction in the state of (B), a tool portion of the end-effector 200 is raised upward in the state of (C), and the tool portion is lowered downward in the state of (D), thereby generating the pitching operation.

According to the above description, the operation by the first actuator 110 occurs with respect to the mounting plate 115 disposed above the first plate 111a, and the operations by the second actuator 120 and the third actuator 130 occur with respect to the first plate 111a. Here, the second actuator 120 causes the yawing operation with respect to the first plate 111a, and the third actuator 130 operates the entire structure of the first actuator 110 including the first plate 111a.

Accordingly, the operation by the third actuator 130 simultaneously appear on the first plate 111a separately from the operation of the first plate 111a by the second actuator 120, and thus, the first plate 111a exhibits a combined operation due to the operations of the second actuator 120 and the third actuator 130, that is, a combination of the yawing operation by the second actuator 120 and the vertical elevating and the pitching operations by the third actuator 130.

This combined operation appears as it is on the mounting plate 115 placed thereabove. At this time, the mounting plate 115 performs the combined movement by the first plate 111a, and also separately performs the shifting operation on the first plate 111a by the first actuator 110.

Thus, the mounting plate 115 may cause the combined operation of four degrees of freedom including all operations described above including the shifting operation by the first actuator 110, the yawing operation by the second actuator 120, and the vertical rising and falling (bouncing) operation and the pitching operation by the third actuator 130.

As a result, the surgical tool 220 mounted on the mounting plate 115 generating the combined motions of four degrees of freedom, may become capable of controlling the combined motions of four degrees of freedom.

The surgical device capable of controlling the operations by the tool of four degrees of freedom may be used in a computer-based surgical system based on a surgical plan. Thus, it is possible to control all of the actuators described above, by using the surgical system, and thus, a surgeon may relatively more freely perform surgery according to a surgical plan, with less physical restrictions and surgical space limitations.

Therefore, according to the surgical device according to the present disclosure, surgery having a high degree of freedom may be possible, whereby a surgeon may have a relatively more free posture, and an end-effector may be operated to correspond to a position and a direction of a corresponding surgical site based on a pre-set surgical plan.

Although various embodiments of the present disclosure have been described in detail above, one of ordinary skill in the art may implement the present disclosure by modifying the present disclosure in various ways without deviating from the concept and the range of the present disclosure defined in the accompanying claims. Therefore, future changes in the embodiments of the present disclosure shall not deviate from the description of the present disclosure.

## Claims

1. A surgical device comprising: a mounting plate on which an end-effector comprising a surgical tool extending in a first direction is mounted, the mounting plate being placed on a plane parallel to both a first axis in the first direction and a second axis in a second direction, the second direction crossing the first direction;
a first actuator on which the mounting plate is mounted and which is configured to cause a first motion of the mounting plate in the second direction;
a second actuator configured to generate rotational force to rotate the first actuator about a third axis in a third direction orthogonal to the first axis and the second axis and to cause a second motion of the mounting plate; and
a third actuator coupled to the second actuator and configured to transmit the rotational force from the second actuator to the first actuator and to cause a third motion of the mounting plate by rotating the mounting plate around a rotation axis in the second direction.

2. The surgical device of claim 1, wherein the first actuator includes: a first plate on which the mounting plate is installed to be reciprocally movable in the second direction; a first motor configured to drive the mounting plate; and a first power transmission unit configured to transmit power of the first motor to operate the mounting plate.

3. The surgical device of claim 2, wherein the first power transmission unit includes: a first conversion unit configured to convert rotation of the first motor into a linear reciprocating movement; and a first operating rod configured to transmit a linear movement from the first conversion unit as a linear movement of the mounting plate.

4. The surgical device of claim 3, wherein the linear movement from the first conversion unit occurs in a direction crossing a plane of the mounting plate, and the first operating rod is hinge-coupled to the mounting plate to convert the linear movement from the first conversion unit into the movement of the mounting plate.

5. The surgical device of claim 3, wherein the first conversion unit includes a first linear mover configured to perform the linear reciprocating movement by rotation of the first motor, and the first operating rod is hinge-coupled to the mounting plate and the first linear mover.

6. The surgical device of claim 1, wherein the first, second, and third actuators are disposed within a housing that protects the first, second, and third actuators, and the second actuator includes: a second plate fixed in position with respect to the housing; a second motor configured to generate rotational force to rotate the first actuator; and a second motor mount configured to support the second motor.

7. The surgical device of claim 6, wherein the third actuator includes a third plate rotated by the second motor.

8. The surgical device of claim 1, wherein the third actuator includes:
a third motor and a fourth motor configured to generate rotational forces to generate a third motion of the mounting plate; and
a second power transmission unit configured to generate the third motion by using respective rotations of the third motor and the fourth motor.

9. The surgical device of claim 8, wherein the second power transmission unit includes:
a second conversion unit and a third conversion unit configured to convert the respective rotations of the third motor and the fourth motor into linear reciprocating movement; and
a third operating rod and a fourth operating rod configured to transmit respective linear movements of the second conversion unit and the third conversion unit to the mounting plate to generate the third motion.

10. The surgical device of claim 9, wherein the third actuator is configured such that the second conversion unit and the third conversion unit of the second power transmission unit move a same distance at a same time to generate a fourth motion in which the mounting plate performs translational movement in the third direction without a change in inclination.

11. A method of controlling a surgical device, the method comprising:
mounting an end-effector having a tool arranged in a first direction on a mounting plate placed on a plane parallel to both a first axis in the first direction and a second axis in a second direction crossing the first direction; causing, by a first actuator, a first motion of the mounting plate in the second direction;
causing, by a second actuator, a second motion of the mounting plate by rotating the first actuator around a third axis in a third direction orthogonal to the first axis and the second axis; and
causing, by a third actuator, a third motion of the mounting plate, the third actuator being coupled to the second actuator and configured to transmit the rotational force from the second actuator to the first actuator and rotate the mounting plate around a rotation axis in the second axis.

12. The method of claim 11, wherein a first plate provided in the first actuator supports the mounting plate to be reciprocally movable in the second direction, and a first power transmission unit provided in the first actuator causes the first motion of the mounting plate.

13. The method of claim 12, wherein a first conversion unit provided in the first power transmission unit converts rotation of the first motor into linear reciprocating movement, and a first operating rod connected to the first conversion unit transmits a linear movement from the first conversion unit to the mounting plate.

14. The method of claim 13, wherein the first conversion unit generates a linear movement in a direction crossing a plane of the mounting plate, and the first operating rod is hinge-coupled to the mounting plate to convert the linear movement from the first conversion unit into the movement of the mounting plate.

15. The method of claim 11, wherein a housing protects the first, second, and
third actuators provided within the housing, and
a second plate provided in the second actuator is fixed in position with respect to the housing and supports the first, second, and third actuators as a whole.

16. The method of claim 11, wherein the third actuator transmits a rotational operation from the second actuator to the mounting plate to cause the second motion, and causes the third motion by using a second conversion unit and a third conversion unit that convert respective rotations of a third motor and a fourth motor into linear reciprocating movement.

17. The method of claim 16, wherein the third actuator generates a fourth motion, in which the mounting plate performs translational movement in the third direction without a change in inclination, by using same-distance movements of the second conversion unit and the third conversion unit.

## Amended claims

### Amended claims under Art. 19.1 PCT

1. A surgical device comprising: a mounting plate on which an end-effector comprising a surgical tool extending in a first direction is mounted, the mounting plate being placed on a plane parallel to both a first axis in the first direction and a second axis in a second direction, the second direction crossing the first direction;
a first actuator on which the mounting plate is mounted and which is configured to cause a first motion of the mounting plate in the second direction;
a second actuator configured to generate rotational force to rotate the first actuator about a third axis in a third direction orthogonal to the first axis and the second axis and to cause a second motion of the mounting plate; and
a third actuator coupled to the second actuator and configured to transmit the rotational force from the second actuator to the first actuator and to cause a third motion of the mounting plate by rotating the mounting plate around a rotation axis in the second direction.

2. The surgical device of claim 1, wherein the first actuator includes: a first plate on which the mounting plate is installed to be reciprocally movable in the second direction; a first motor configured to drive the mounting plate; and a first power transmission unit configured to transmit power of the first motor to operate the mounting plate.

3. The surgical device of claim 2, wherein the first power transmission unit includes: a first conversion unit configured to convert rotation of the first motor into a linear reciprocating movement; and a first operating rod configured to transmit a linear movement from the first conversion unit as a linear movement of the mounting plate.

4. The surgical device of claim 3, wherein the linear movement from the first conversion unit occurs in a direction crossing a plane of the mounting plate, and the first operating rod is hinge-coupled to the mounting plate to convert the linear movement from the first conversion unit into the movement of the mounting plate.

5. The surgical device of claim 3, wherein the first conversion unit includes a first linear mover configured to perform the linear reciprocating movement by rotation of the first motor, and the first operating rod is hinge-coupled to the mounting plate and the first linear mover.

6. The surgical device of claim 1, wherein the first, second, and third actuators are disposed within a housing that protects the first, second, and third actuators, and the second actuator includes: a second plate fixed in position with respect to the housing; a second motor configured to generate rotational force to rotate the first actuator; and a second motor mount configured to support the second motor.

7. The surgical device of claim 6, wherein the third actuator includes a third plate rotated by the second motor.

8. The surgical device of claim 1, wherein the third actuator includes:
a third motor and a fourth motor configured to generate rotational forces to generate a third motion of the mounting plate; and
a second power transmission unit configured to generate the third motion by using respective rotations of the third motor and the fourth motor.

9. The surgical device of claim 8, wherein the second power transmission unit includes:
a second conversion unit and a third conversion unit configured to convert the respective rotations of the third motor and the fourth motor into linear reciprocating movement; and
a third operating rod and a fourth operating rod configured to transmit respective linear movements of the second conversion unit and the third conversion unit to the mounting plate to generate the third motion.

10. The surgical device of claim 9, wherein the third actuator is configured such that the second conversion unit and the third conversion unit of the second power transmission unit move a same distance at a same time to generate a fourth motion in which the mounting plate performs translational movement in the third direction without a change in inclination.

11. A method of controlling a surgical device, the method comprising:
mounting an end-effector having a tool arranged in a first direction on a mounting plate placed on a plane parallel to both a first axis in the first direction and a second axis in a second direction crossing the first direction;
causing, by a first actuator, a first motion of the mounting plate in the second direction;
causing, by a second actuator, a second motion of the mounting plate by rotating the first actuator around a third axis in a third direction orthogonal to the first axis and the second axis; and
causing, by a third actuator, a third motion of the mounting plate, the third actuator being coupled to the second actuator and configured to transmit a rotational force from the second actuator to the first actuator and rotate the mounting plate around a rotation axis in the second axis.

12. The method of claim 11, wherein a first plate provided in the first actuator supports the mounting plate to be reciprocally movable in the second direction, and a first power transmission unit provided in the first actuator causes the first motion of the mounting plate.

13. The method of claim 12, wherein a first conversion unit provided in the first power transmission unit converts rotation of a first motor into linear reciprocating movement, and a first operating rod connected to the first conversion unit transmits a linear movement from the first conversion unit to the mounting plate.

14. The method of claim 13, wherein the first conversion unit generates a linear movement in a direction crossing a plane of the mounting plate, and the first operating rod is hinge-coupled to the mounting plate to convert the linear movement from the first conversion unit into the movement of the mounting plate.

15. The method of claim 11, wherein a housing protects the first, second, and
third actuators provided within the housing, and
a second plate provided in the second actuator is fixed in position with respect to the housing and supports the first, second, and third actuators as a whole.

16. The method of claim 11, wherein the third actuator transmits a rotational operation from the second actuator to the mounting plate to cause the second motion, and causes the third motion by using a second conversion unit and a third conversion unit that convert respective rotations of a third motor and a fourth motor into linear reciprocating movement.

17. The method of claim 16, wherein the third actuator generates a fourth motion, in which the mounting plate performs translational movement in the third direction without a change in inclination, by using same-distance movements of the second conversion unit and the third conversion unit.
